(19) Europäisches Patentamt
European Patent Office
Office européen des brevets

(11) Publication number: **0 304 293 B1**

(12) # EUROPEAN PATENT SPECIFICATION

(45) Date of publication of patent specification: **17.03.93** (51) Int. Cl.[5]: **C12P 7/62**, C08G 63/06

(21) Application number: **88307635.8**

(22) Date of filing: **17.08.88**

(54) **Copolyester and process for producing the same.**

(30) Priority: **18.08.87 JP 204537/87**
**18.08.87 JP 204538/87**
**15.12.87 JP 316446/87**
**02.03.88 JP 49015/88**
**18.07.88 JP 178448/88**

(43) Date of publication of application:
**22.02.89 Bulletin 89/08**

(45) Publication of the grant of the patent:
**17.03.93 Bulletin 93/11**

(84) Designated Contracting States:
**DE GB IT**

(56) References cited:
**EP-A- 0 069 497**
**EP-A- 0 204 442**

**DIE MAKROMOLEKULARE CHEMIE, vol. 8, no. 12, December 1987, pages 631-635, Basel, CH; Y. DOI et al.: "Biosynthesis of terpolyesters of 3-hydroxybutyrate, 3-hydroxyvalerate, and 5-hydroxyvalerate in Alcaligenes eutrophus from 5-chloropentanoic and pentanoic acids"**

**ENVIRONMENTAL SCIENCE & TECHNOLOGY, vol. 8, no. 6, June 1974, pages 576-579; L.L. WALLEN et al.: "Poly-beta-hydroxyalkanoate from activated sludge"**

(73) Proprietor: **MITSUBISHI KASEI CORPORATION**
**5-2, Marunouchi 2-chome Chiyoda-ku**
**Tokyo 100(JP)**

(72) Inventor: **Doi, Yoshiharu**
**2617-39 Imajuku-cho Asahi-ku**
**Yokohama-shi Kanagawa-ken(JP)**

(74) Representative: **Woods, Geoffrey Corlett et al**
**J.A. KEMP & CO. 14 South Square Gray's Inn**
**London WC1R 5EU (GB)**

**EP 0 304 293 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a copolyester comprising 3-hydroxybutyrate units and to a process for the production thereof.

Poly-3-hydroxybutyrate (hereinafter referred to as PHB) is a thermoplastic high polymer which is accumulated as the energy-storage substance in the cells of a large number of microorganisms. It has excellent biodegradability and excellent adaptability to living bodies. It has attracted attention as a "clean" plastic which does not harm the environment. Its application in various fields, for example as a biomedical material such as a suture material or a fixation material for bone fracture or in a slow-release system which slowly releases a medicine or agricultural chemical has been expected for many years. Particularly in recent years, since synthetic plastics present severe social problems due to environmental pollution and problems of non-renewable sources, PHB has attracted attention as a biopolymer which does not depend on petroleum.

However, since PHB has poor impact strength due to its rigidity, it is not suitable for practical use. The cost of producing PHB is high. It has therefore not been industrially produced. In order to improve the impact strength of PHB, it has been proposed to produce a copolymer of 3-hydroxybutyrate.

For instance JP-A-57-150393 (1982) and JP-A-59-220192 (1984) disclose a copolymer comprising 3-hydroxybutyrate and 3-hydroxyvalerate units and a process for producing the copolymer which comprises propagating a microorganism as in the conventional process for producing PHB and subsequently culturing the microorganism in a limited source of nitrogen and/or phosphorus.

JP-A-57-150393 (1982) indicates that a copolyester comprising from 99.5 to 50 mol% of 3-hydroxybutyrate units and from 0.1 to 50 mol% of other ester units such as 3-hydroxyvalerate units can be produced by using propionic acid and isobutyric acid as the culture substrate. However only a copolyester containing at most 33 mol% of 3-hydroxyvalerate units is disclosed.

JP-A-59-220192 (1984) indicates that a copolyester comprising at least 40 mol% of 3-hydroxybutyrate units and other ester units can be produced using carbon from the cellular substance of the wasted microorganism after extracting PHB in the latter stage of culture. The exact ratio of 3-hydroxyvalerate and 3-hydroxybutyrate units is not described. The process disclosed is complicated and the composition of the cellular substance varies in kind and amount according to the culture conditions. Thus the process is not practical.

When the content of 3-hydroxyvalerate units in the copolyester increases from 0 to 33 mol%, the melting point (Tm) of the copolyester is rapidly lowered from 180°C to 85°C [refer to T L Bluhm et al, Macromolecules, 19,2871-2876 (1986). This dependency of the melting point on the content of the 3-hydroxyvalerate units means that it is difficult to obtain industrially a product of consistent quality.

We have sought an industrially profitable and easy method for producing a copolyester in which the molar content of other units is relatively large when compared with the molar content of 3-hydroxybutyrate units.

The present invention provides a copolyester comprising 3-hydroxybutyrate (3HB) units and units selected from:

(1) 3-hydroxyvalerate (3HV) units and 5-hydroxyvalerate (5HV) units;
(2) 4-hydroxybutyrate (4HB) units; and
(3) 4-hydroxybutyrate (4HB) units and 3-hydroxyvalerate (3HV) units;

the intrinsic viscosity of the copolyester being from 0.4 to 10.0 dl/g when measured in chloroform at 30°C.

The present invention also provides a process for producing a copolyester as defined above, which process comprises:

culturing Alcaligenes eutrophus in a limited source of nitrogen and/or phosphorus and in the presence of a carbon source to form and accumulate the copolyester in the Alcaligenes eutrophus, the carbon source being selected from:

(i) a compound of formula (I):

$$(CH_2X^1CH_2CH_2CH_2COO)_{n^1}Y^1 \quad (I)$$

wherein $X^1$ represents a hydroxyl group or a halogen atom, which may be identical or different to any other $X^1$ group if $n^1$ is greater than 1, $n^1$ represents an integer of from 1 to 4 and $Y^1$ represents a hydrogen atom or a uni- to tetravalent metal atom;

(ii) a compound of formula (II):

$$(CH_2X^2CH_2CH_2COO)_{n^2}Y^2 \quad (II)$$

wherein $X^2$ represents a hydroxyl group or a halogen atom, which may be identical or different to any other $X^2$ group if $n^2$ is greater than 1, $n^2$ represents an integer of from 1 to 4 and $Y^2$ represents a hydrogen atom or a uni- to tetravalent metal atom;

(iii) a compound of formula (II) as defined above and a compound of formula (III):

$$(CH_2X^3CHX^4CH_2CH_2COO)_{n^3}Y^3 \quad (III)$$

wherein $X^3$ represents a hydrogen atom, a halogen atom or a hydroxyl group, which may be identical or different to any other $X^3$ group if $n^3$ is greater than 1, $X^4$ represents a hydrogen atom, a halogen atom, a hydroxyl group or an alkyl group, $n^3$ represents an integer of from 1 to 4 and $Y^3$ represents a hydrogen atom or a uni- to tetravalent metal atom;

(iv) 1,4-butanediol; and

(v) $\gamma$-butyrolactone.

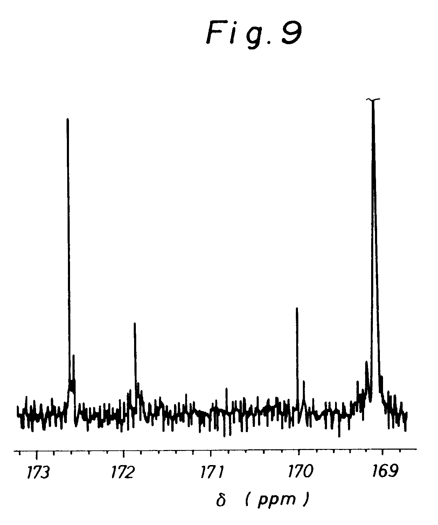

In the attached drawings, Figs. 1, 3, 5 and 7 respectively show the $^1$H-NMR spectrum at 500 MHz of the copolyesters obtained in Examples 2, 7, 16 and 21, and Figs. 2, 4, 6 and 8 respectively show the $^{13}$C-NMR spectrum at 125 MHz of the copolyesters obtained in Examples 2, 7, 16 and 21. Fig. 9 is an enlarged spectrum of part of the spectrum of Fig. 8. The numerical references of the peaks of Figs. 1 to 4 correspond to the numerical references of the following formulae:

Fig. 1:

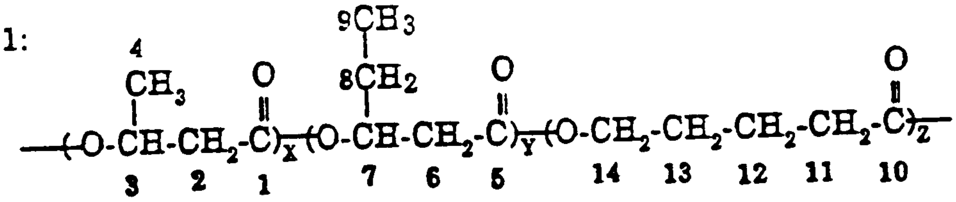

3HB 3HV 5HV

Fig. 2:

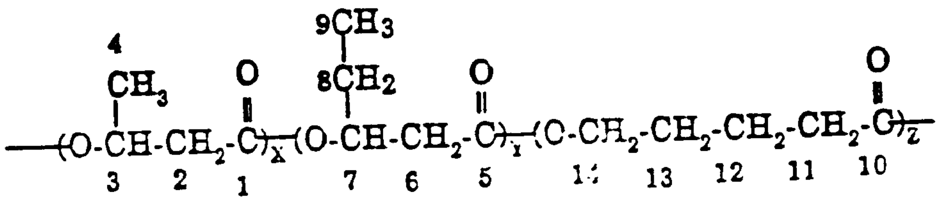

3HB 3HV 5HV

Fig. 3:

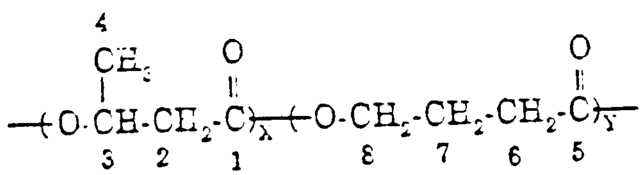

3HB 4HB

Fig. 4:

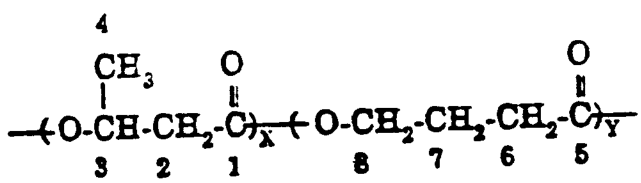

3HB 4HB

The 3HB, 4HB, 3HV and 5HV units contained in the copolyester of the present invention are of the

following formulae:

3 HB unit: $-OCH(CH_3)CH_2\underset{\underset{O}{\|}}{C}-$

4 HB unit: $-OCH_2CH_2CH_2\underset{\underset{O}{\|}}{C}-$

3 HV unit: $-OCH(C_2H_5)CH_2\underset{\underset{O}{\|}}{C}-$ and

5 HV unit: $-OCH_2CH_2CH_2CH_2\underset{\underset{O}{\|}}{C}-$

Examples of suitable strains of Alcaligenes eutrophus are Alcaligenes eutrophus H-16 ATCC 17699, Alcaligenes eutrophus NCIB 11597 which is a mutant of the H-16 strain, Alcaligenes eutrophus NCIB 11598, Alcaligenes eutrophus NCIB 11599 and Alcaligenes eutrophus NCIB 11600. Of these strains, Alcaligenes eutrophus H-16 ATCC 17699 and Alcaligenes eutrophus NCIB 11599 are particularly preferred in practical use.

The microbiological characteristics of microorganisms of the genus Alcaligenes are described in, for instance, BERGEY'S MANUAL OF DETERMINATIVE BACTERIOLOGY: Eighth Edition, The Williams & Wilkins Company/Baltimore, and the microbiological characteristics of Alcaligenes eutrophus H-16 are described in, for instance, J. Gen. Microbiol., 115, 185 - 192(1979).

These microorganisms are cultured in two stages as in the conventional process; a former stage in which the microorganism is mainly propagated and a latter stage in which the copolyester is formed and accumulated in the microorganism under limitation of nitrogen and/or phosphorus.

In the former stage a conventional culture process can be used for propagation. Thus a conventional culture medium and culture conditions may be employed.

The components of the culture medium are not particularly limited if they are substances which can be used as the substrate by the microorganism. In practical use the carbon source is selected from, for example, synthetic carbon sources such as methanol, ethanol and acetic acid; inorganic carbon sources such as carbon dioxide; natural substances such as yeast extract, molasses, peptone and meat extract; sugars such as arabinose, glucose, mannose, fructose and galactose; and sugar alcohols such as sorbitol, mannitol and inositol. The nitrogen source is selected from, for example, inorganic nitrogen compounds such as ammonia, ammonium salts and nitrates; and/or organic nitrogen compounds such as urea, corn steep liquor, casein, peptone, yeast extract and meat extract. The inorganic component is selected from, for example, calcium salts, magnesium salts, potassium salts, sodium salts, phosphates, manganese salts, zinc salts, iron salts, copper salts, molybdenum salts, cobalt salts, nickel salts, chromium salts, boron compounds and iodine compounds.

Furthermore, as occasion demands, vitamins may be used.

The culture temperature is, for instance, from 20 to 40 °C, preferably from 25 to 35 °C and the pH of the culture medium is, for instance, from 6 to 10, preferably from 6.5 to 9.5. The culture is carried out aerobically under these conditions.

The culture may be carried out under different conditions from the above, even though the propagation of the microorganism may be relatively inefficient, if the microorganism is practically propagated.

The mode of culture may be either batch culture or continuous culture.

The microorganism propagated in the former stage of culture is further cultured under conditions of limited nitrogen and/or phosphorus.

Thus the microorganisms are separated and collected from the culture liquid of the former stage of culture by a conventional liquid-solid separation technique such as filtration and centrifugation, and the collected microorganisms are subjected to the latter stage of culture. Instead of the above method, a method in which nitrogen, phosphorus or both are substantially exhausted after the former stage of culture and the culture liquid is subjected to the latter stage of culture without separating and collecting the microorganisms may be also employed.

In the latter stage of culture, the procedures are not necessarily different from the procedures in the former stage of culture except that the culture medium or liquid contains substantially no nitrogen and/or phosphorus and includes a carbon source selected from (1) a compound of formula (I), (2) a compound of formula (II), (3) a compound of formula (II) and a compound of formula (III), (4) 1,4-butanediol and (5) γ-butyrolactone.

In formula (I), $X^1$ represents a hydroxyl group or a halogen atom. As the halogen atom, a chlorine atom or a bromine atom is preferred. $Y^1$ represents a hydrogen atom, a univalent metal atom such as sodium and potassium, a divalent metal atom such as calcium and magnesium, a trivalent metal atom such as aluminium or a tetravalent metal atom. Among these metals, uni- to trivalent metals are preferred. Furthermore $n^1$ represents an integer of from 1 to 4. Examples of compounds of formula (I) are 5-chlorovaleric acid, 5-hydroxyvaleric acid, a sodium salt thereof, a potassium salt thereof and a calcium salt thereof.

In formula (II), $X^2$ represents a hydroxyl group or a halogen atom. As the halogen atom, a chlorine atom or a bromine atom is preferred. $Y^2$ represents a hydrogen atom, a univalent metal atom such as sodium and potassium, a divalent metal atom such as calcium and magnesium, a trivalent metal atom such as aluminium, or a tetravalent metal atom. Among these metals, uni- to trivalent metals are preferred. Furthermore $n^2$ represents an integer of from 1 to 4.

Examples of compounds of formula (II) are derivatives of butyric acid such as 4-hydroxybutyric acid, 4-chlorobutyric acid and 4-bromobutyric acid, a sodium salt thereof, a potassium salt thereof, a magnesium salt thereof, a calcium salt thereof and an aluminium salt thereof.

In formula (III), $X^3$ represents a hydrogen atom, a hydroxyl group or a halogen atom. As the halogen atom, a chlorine atom and a bromine atom are preferred. $X^4$ represents a hydrogen atom, a halogen atom, preferably a chlorine atom or a bromine atom, a hydroxyl group or an alkyl group, preferably an alkyl group of from 1 to 3 carbon atoms. $Y^3$ represents a hydrogen atom, a univalent metal atom such as sodium and potassium, a divalent metal atom such as calcium and magnesium, a trivalent metal atom such as aluminium, or a tetravalent metal atom. Furthermore $n^3$ represents an integer of from 1 to 4.

Examples of compounds of formula (III) are valeric acid, 4-chlorovaleric acid, 4-hydroxyvaleric acid, 4-methylvaleric acid, 4-ethylvaleric acid, 5-hydroxyvaleric acid, 5-chlorovaleric acid, sodium salts thereof and potassium salts.

The compound of formula (I), the compound of formula (II), a mixture of the compounds of formulae (II) and (III), 1,4-butanediol or γ-butyrolactone is present in the culture medium or the culture liquid in the latter stage of culture. The above substances may be present at any time during the latter stage of culture from the beginning to the end of the culture. However, the substances are preferably present at the beginning of the latter stage of culture.

The compound of formula (I) or (II) or 1,4-butandiol may be used in an amount which can form the desired copolyester and which does not hinder the growth of the microorganisms. Although the amount depends on the strain of the microorganism used and the molar ratios of the constituent units in the desired copolyester, the above compounds are generally present in an amount of from 3 to 40 g, preferably from 5 to 30 g, per litre of the culture medium or the culture liquid.

When using the compound of formula (II) together with the compound of formula (III), the total amount of the compounds is preferably from 3 to 40 g, more preferably from 5 to 30 g, per litre of the culture medium or the culture liquid. The weight proportion of the compound of formula (II) is preferably from 30 to 95%, more preferably from 35 to 90%, and the weight proportion of the compound of formula (III) is preferably from 5 to 70%, more preferably from 10 to 65%.

When using γ-butyrolactone, the amount of γ-butyrolactone is preferably from 3 to 100 g, more preferably from 5 to 90 g, per litre of the culture medium or the culture liquid.

In the latter stage of culture, the compound of formula (I), the compound of formula (II) a mixture of the compounds of formulae (II) and (III), 1,4-butanediol or γ-lactone must be used as the carbon source. However, another carbon source which can be used as the substrate by the microorganism, for instance glucose, fructose, methanol, ethanol, acetic acid, propionic acid, n-butyric acid, lactic acid, valeric acid and a salt of these acids, can be used together. For instance, in the case of using glucose, the amount of glucose is at most 1.5 g/litre.

When using γ-butyrolactone, 4-hydroxybutyric acid, a salt thereof and a diol such as 1,4-butanediol may be used together with the γ-butyrolactone.

The microorganisms are separated and collected from the culture liquid or the culture medium obtained by the above culture by a conventional liquid-solid separation technique such as filtration and centrifugation. The collected microorganisms are washed and dried to obtain the dried fungus. From the dried fungus the copolyester formed therein is extracted by a known method, for instance extraction with an organic solvent such as chloroform. The copolyester is precipitated by adding a poor solvent, for instance hexane, to the thus obtained extract. The copolyester of the present invention can be obtained by an ordinary method of separation.

According to the process of the present invention, when a compound of formula (I) is used in the latter stage of culture, a copolyester comprising from 2 to 50 mol%, preferably from 10 to 40 mol%, of the 3HB unit, from 3 to 95 mol%, preferably from 10 to 90 mol%, of the 3HV unit and from 3 to 90 mol%, preferably from 5 to 80 mol%, of the 5HV unit can be obtained.

When the compound of formula (II), 1,4-butanediol or γ-butyrolactone is used, a copolyester comprising from 40 to 97 mol%, preferably from 50 to 95 mol%, of the 3HB unit and from 3 to 60 mol%, preferably from 5 to 50 mol%, of the 4HB unit can be obtained.

When the compound of formula (II) is used together with the compound of formula (III), a copolyester comprising from 10 to 90 mol%, preferably from 10 to 80 mol%, of the 3HB unit, from 3 to 60 mol%, preferably from 3 to 50 mol%, of the 4HB unit and from 5 to 87 mol%, preferably from 10 to 70 mol%, of the 3HV unit can be obtained.

When the proportion of the 3HB unit in the copolyester of the present invention is not larger than 50 mol%, the stability of the melting temperature is greater and the crystallization degree is smaller. Accordingly the copolyester has excellent strength and the formation thereof, such as by spinning and rolling, is easier and stabilized. The formed products such as fibers and films are flexible and tough.

The present invention is now further explained in the following Examples.

EXAMPLES 1 TO 3 AND COMPARATIVE EXAMPLE 1:

Copolyesters were produced by using Alcaligenes eutrophus NCIB 11599 as follows.

Former stage of culture

The microorganism was cultured in a culture medium having the following composition for 24 hours at 30°C, and the microorganisms were separated from the culture liquid at the termination of the logarithmic propagation phase by centrifugation.

| Composition of the culture medium | | | |
|---|---|---|---|
| Yeast extract | 10 g | Polypeptone | 10 g |
| Meat extract | 5 g | $(NH_4)_2SO_4$ | 5 g |

The above substances were dissolved in one litre of deionized water, and the pH of the solution was adjusted to 7.0.

Latter stage of culture

The microorganisms obtained in the former stage of culture were suspended in a culture medium having the following composition in an amount of 5 g/litre and were cultured for 48 hours at 30°C.

The microorganisms were separated by centrifugation from the thus obtained culture liquid.

**Composition of the culture medium**

0.5 M aqueous solution of potassium dihydrogenphosphate 39.0 ml

0.5 M aqueous solution of dipotassium dihydrogenphosphate 53.6 ml

20 Wt/V% aqueous solution of magnesium sulfate 1.0 ml

Carbon source*

A mineral solution**

*As the carbon source, 5-chlorovaleric acid and valeric acid were used (unit: g/litre of medium) as shown in TABLE 1.

In COMPARATIVE EXAMPLE 1, 20 g of butyric acid were used.

**A mineral solution

| | |
|---|---|
| $CoCl_2$ | 119.0 mg |
| $FeCl_3$ | 9.7 g |
| $CaCl_2$ | 7.8 g |
| $NiCl_2$ | 118.0 mg |
| $CrCl_2$ | 62.2 mg |
| $CaSO_4$ | 156.4 mg |

These salts were dissolved in one litre of 0.1M hydrogen chloride.

Treatment of the microorganism

The microorganisms obtained in the latter stage of culture were washed with distilled water and acetone, and then dried under reduced pressure (0.1mm Hg at 20 ° C) to obtain the dried fungus.

Separation and collection of the copolyester

The copolyester was extracted from the dried fungus with hot chloroform, and hexane was added to the extract to precipitate the copolyester. The precipitate was collected by filtration and dried to obtain the copolyester.

Properties of the copolyester

The composition, the intrinsic viscosity, the melting temperature and the heat of fusion of the copolyester were measured as follows:

| | |
|---|---|
| Composition: | by $^1$H-NMR at 500 MHz |
| Intrinsic viscosity [$\eta$]: | in chloroform at 30 ° C |
| Melting temperature Tm: | by DSC determination at a rate of temperature increase of 10 ° C/min |
| Heat of fusion $\Delta$H: | by DSC determination |

The results are shown in TABLE 1.

The $^{1}H$-NMR spectrum at 500 MHz of the copolyester obtained in EXAMPLE 2 is shown in Fig. 1, and the $^{13}C$-NMR spectrum at 125 MHz of the same is shown in Fig. 2.

TABLE 1

| | Carbon source(g) | | Weight of dried fungus (g) | Content of copolyester (%) | Composition of copolyester (mol%) | | | [η] (dl/g) | Melting temperature (°C) | Heat of fusion (cal/g) (J/g) |
|---|---|---|---|---|---|---|---|---|---|---|
| | 5-chloro-valeric acid | valeric acid | | | [3HB] | [3HV] | [5HV] | | | |
| Example 1 | 20 | 0 | 4.7 | 1 | 24 | 24 | 52 | - | - | - |
| Example 2 | 10 | 10 | 4.4 | 8 | 26 | 63 | 11 | 3.1 | 101 | 11.2 (46.9) |
| Example 3 | 5 | 15 | 4.2 | 19 | 26 | 65 | 9 | 5.0 | - | - |
| Comparative Example 1 | - | - | 9.4 | 51 | 100 | 0 | 0 | 3.3 | 177 | 19.5 (81.6) |

EXAMPLES 4 to 12 AND COMPARATIVE EXAMPLE 2:

Copolyesters were obtained as in EXAMPLE 1 except for using Alcaligenes eutrophus H-16 ATCC 17699 and also using the compounds shown in TABLE 2 as the carbon source in the latter stage of culture. The composition and the intrinsic viscosity of the copolyesters obtained were measured as in EXAMPLE 1, and the results are shown in TABLE 2.

The $^{1}H$-NMR spectrum at 500 MHz of the copolyester obtained in EXAMPLE 7 is shown in Fig. 3 and the $^{13}C$-NMR spectrum at 125 MHz of the same is shown in Fig. 4.

TABLE 2

| | Carbon source (g) | | Weight of dried fungus (g) | Content of copolyester (%) | Composition of copolyester (mol%) | | [η] (dl/g) |
|---|---|---|---|---|---|---|---|
| | 4-hydroxy butyric acid | butyric acid | | | 3HB | 4HB | |
| Example 4 | 4 | 0 | 2.8 | 7 | 75 | 25 | - |
| Example 5 | 8 | 0 | 3.3 | 14 | 74 | 26 | - |
| Example 6 | 12 | 0 | 4.1 | 18 | 74 | 26 | - |
| Example 7 | 16 | 0 | 3.5 | 19 | 73 | 27 | 4.3 |
| Example 8 | 20 | 0 | 2.9 | 19 | 69 | 31 | - |
| Example 9 | 24 | 0 | 3.5 | 13 | 66 | 34 | 4.0 |
| Example 10 | 28 | 0 | 3.5 | 8 | 64 | 36 | - |
| Example 11 | 4 | 15 | 8.5 | 53 | 95 | 5 | - |
| Example 12 | 8 | 10 | 7.6 | 48 | 87 | 13 | 3.9 |
| Comparative Example 2 | 0 | 20 | 9.6 | 51 | 100 | 0 | 3.3 |

EXAMPLE 13:

The properties the copolyester obtained by the same procedures as in EXAMPLE 4 except for using 4-chlorobutyric acid (18 g/litre) as the carbon source in the latter stage of culture are shown in TABLE 3.

Of the data in TABLE 3, the chain distribution, the melting temperature and the heat of fusion of the copolyester were measured as follows:

Chain distribution; estimated from the multiplet resonance structure of the carbonyl carbon according to the method of the present inventor et al. (Y. Doi et al., Macromolecules, 19, 2860-2864 (1986))

Melting temperature; by DSC determination (at a rate of temperature increase of 10°C/min).

Heat of fusion; by DSC determination

The calculated values of $C_4H_6O_2$ in the elementary analysis are as follows:

| C | H |
|---|---|
| 55.81 % | 7.02 % |

EXAMPLE 14:

The same procedure as in EXAMPLE 13 was repeated except for using sodium 4-hydroxybutyrate (20 g/litre) as the carbon source in the latter stage of culture. The results are shown in TABLE 3.

EXAMPLE 15:

The same procedure as in EXAMPLE 10 was repeated except for using Alcaligenes eutrophus NCIB 11599. The results are shown in TABLE 3.

TABLE 3

| | Weight of dried fungus (g) | Content of copolyester (%) | Composition of copolyester (mol%) | | Chain distribution (mol%) | | | | [η] (dl/g) | Melting temperature (°C) | Heat of fusion (cal/g) (J/g) | Elementary analysis (%) | | |
|---|---|---|---|---|---|---|---|---|---|---|---|---|---|---|
| | | | 3HB | 4HB | [3HB-3HB] | [3HB-4HB] | [4HB-3HB] | [4HB-4HB] | | | | C | H | Cl |
| Example 13 | 5.1 | 27 | 89 | 11 | 82 | 9 | 9 | 0 | 3.9 | 156 | 11.1 (46.5) | 55.50 | 7.09 | 0.29 |
| Example 14 | 3.7 | 30 | 67 | 33 | 55 | 13 | 13 | 19 | 2.9 | 166 | 3.7 (15.5) | 55.60 | 6.64 | - |
| Example 15 | 4.5 | 20 | 51 | 49 | 32 | 21 | 19 | 28 | 6.1 | 159 | 0.5 (2.1) | 55.18 | 6.95 | - |
| Comparative Example 1 | 9.5 | 51 | 100 | - | 100 | 0 | 0 | 0 | 3.3 | 177 | 19.5 (81.6) | 55.88 | 7.34 | - |

EXAMPLES 16 TO 19 AND COMPARATIVE EXAMPLES 3 TO 4:

By the same procedure as in EXAMPLE 1 except for using the compounds shown in TABLE 4 as the carbon source in the latter stage of culture, copolyesters were produced and the properties thereof were

measured. The results are shown in TABLE 4. The $^{1}$H-NMR spectrum at 500 MHz of the copolyester obtained in EXAMPLE 16 is shown in Fig. 5, and the $^{13}$C-NMR spectrum at 125 MHz of the same is shown in Fig. 6.

TABLE 4

| | Carbon source (g) | | Weight of dried fungus (g) | Content of copolyester (% by weight) | Composition of copolyester (mol%) | | | [η] (dl/g) | Melting temperature (°C) | Heat of fusion (cal/g) (J/g) |
|---|---|---|---|---|---|---|---|---|---|---|
| | 4-hydroxy butyric acid | valeric acid | | | [3HB] | [4HB] | [3HV] | | | |
| Example 16 | 17.5 | 2.5 | 7.8 | 18 | 32 | 45 | 23 | 3.7 | 166<br>87<br>78 | 3.1 (13.0)<br>2.3 (9.6) |
| Example 17 | 15.0 | 5.0 | 9.6 | 17 | 34 | 30 | 36 | 5.7 | 163<br>89<br>81 | 2.5 (10.5)<br>3.3 (13.9) |
| Example 18 | 10.0 | 10.0 | 10.4 | 24 | 31 | 14 | 55 | 6.4 | 162<br>92 | 1.6 (6.7)<br>4.8 (20.1) |
| Example 19 | 7.5 | 12.5 | 10.5 | 24 | 28 | 5 | 67 | 7.1 | 171<br>94 | 1.4 (5.8)<br>8.1 (33.9) |
| Comparative Example 3 | 20 | 0 | 7.2 | 19 | 66 | 34 | 0 | 4.0 | 166 | 7.1 (29.7) |
| Comparative Example 4 | 0 | 20 | 12.5 | 36 | 10 | 0 | 90 | 4.0 | 108 | 13.8 (57.8) |

EXAMPLES 20 TO 24 AND COMPARATIVE EXAMPLES 5 TO 7:

By the same procedure as in EXAMPLE 4 except for using the compounds shown in TABLE 5 as the carbon source in the latter stage of culture, copolyesters were produced, and the properties were measured. The results are shown in TABLE 5.

The $^{1}$H-NMR spectrum at 500 MHz of the copolyester obtained in EXAMPLE 21 is shown in Fig. 7, and the $^{13}$C-NMR spectrum at 125 MHz of the same and an enlarged spectrum thereof are shown in Figs. 8 and

9 respectively.

TABLE 5

| | Carbon source (g) | Weight of dried fungus (g) | Content of copolyester (% by weight) | Composition of copolyester (mol%) | | | [η] (dl/g) |
|---|---|---|---|---|---|---|---|
| | | | | 3HB | 4HB | 3HV | |
| Example 20 | 1,4-butanediol 20 | 2.66 | 8 | 75 | 25 | 0 | - |
| Example 21 | 1,4-butanediol 17<br>Butyric acid 3 | 3.91 | 34 | 83 | 17 | 0 | 2.9 |
| Example 22 | 1,4-butanediol 13<br>Butyric acid 7 | 5.05 | 52 | 93 | 7 | 0 | 2.8 |
| Example 23 | 1,4-butanediol 10<br>Butyric acid 10 | 6.20 | 63 | 97 | 3 | 0 | 3.6 |
| Example 24 | 1,4-butanediol 5<br>Butyric acid 15 | 4.60 | 47 | 99 | 1 | 0 | 2.9 |
| Comparative Example 5 | 1,3-propanediol 20 | 3.70 | 3 | 94 | 0 | 6 | - |
| Comparative Example 6 | 1,5-pentanediol 20 | 3.52 | 4 | 95 | 0 | 5 | - |
| Comparative Example 7 | Butyric acid 20 | 4.80 | 51 | 100 | 0 | 0 | 3.3 |

EXAMPLES 25 TO 29 AND COMPARATIVE EXAMPLES 8 TO 9:

By the same procedure as in EXAMPLE 1 except for using Alcaligenes eutrophus H-16 ATCC 17699 and using the compounds shown in TABLE 6 as the carbon source in the latter stage of culture, copolyesters were produced, and the properties thereof were measured. The results are shown in TABLE 6.

TABLE 6

| | Carbon source (g) | | | Weight of dried fungus (g) | Content of copolyester (% by weight) | Composition of copolyester (mol%) | | [η] (dl/g) | Melting temperature (°C) | Heat of fusion (cal/g) (J/g) |
|---|---|---|---|---|---|---|---|---|---|---|
| | γ-butyrolactone | Butyric acid | 4-Hydroxybutyric acid | | | 3HB | 4HB | | | |
| Example 25 | 2.0 | - | - | 0.81 | 6 | 90 | 10 | 3.3 | 169 | 12 (50) |
| Example 26 | 0.5 | 1.5 | - | 1.24 | 28 | 95 | 5 | 3.4 | 165 | 19 (80) |
| Example 27 | 1.0 | 1.0 | - | 1.03 | 22 | 93 | 7 | - | 165 | 18 (75) |
| Example 28 | 1.5 | 0.5 | - | 0.80 | 8 | 89 | 11 | - | 170 | 13 (54) |
| Example 29 | 10 | 10 | - | 7.50 | 42 | 73 | 27 | 2.7 | 168 | 9 (38) |
| Comparative Example 8 | - | 2.0 | - | 1.58 | 36 | 100 | 0 | 3.3 | 175 | 18 (75) |
| Comparative Example 9 | - | 10 | 10 | 7.90 | 28 | 71 | 29 | 2.7 | 167 | 10 (42) |

## Claims

1. A copolyester comprising 3-hydroxybutyrate units and units selected from:
   (1) 3-hydroxyvalerate units and 5-hydroxyvalerate units;
   (2) 4-hydroxybutyrate units; and
   (3) 4-hydroxybutyrate units and 3-hydroxyvalerate units;
   the intrinsic viscosity of the copolyester when measured in chloroform at 30 °C being from 0.4 to 10.0 dl/g.

2. A copolyester according to claim 1 which comprises from 2 to 50 mol% of 3-hydroxybutyrate units, from 3 to 95 mol% of 3-hydroxyvalerate units and from 3 to 90 mol% of 5-hydroxyvalerate units.

3. A copolyester according to claim 1 which comprises from 40 to 97 mol% of 3-hydroxybutyrate units and from 3 to 60 mol% of 4-hydroxybutyrate units.

4. A copolyester according to claim 1 which comprises from 10 to 90 mol% of 3-hydroxybutyrate units, from 3 to 60 mol% of 4-hydroxybutyrate units and from 5 to 87 mol% of 3-hydroxyvalerate units.

5. A process for producing a copolyester as defined in claim 1, which process comprises:
   culturing Alcaligenes eutrophus in a limited source of nitrogen and/or phosphorus and in the presence of a carbon source to form and accumulate the copolyester in the Alcaligenes eutrophus, the carbon source being selected from:
   (i) a compound of formula (I):

$$(CH_2X^1CH_2CH_2CH_2COO)_{n^1}Y^1 \quad (I)$$

   wherein $X^1$ represents a hydroxyl group or a halogen atom, which may be identical or different to any other $X^1$ group if $n^1$ is greater than 1, $n^1$ represents an integer of from 1 to 4 and $Y^1$ represents a hydrogen atom or a uni- to tetravalent metal atom;
   (ii) a compound of formula (II):

$$(CH_2X^2CH_2CH_2COO)_{n^2}Y^2 \quad (II)$$

   wherein $X^2$ represents a hydroxyl group or a halogen atom, which may be identical or different to any other $X^2$ group if $n^2$ is greater than 1, $n^2$ represents an integer of from 1 to 4 and $Y^2$ represents a hydrogen atom or a uni- to tetravalent metal atom;
   (iii) a compound of formula (II) as defined above and a compound of formula (III):

$$(CH_2X^3CHX^4CH_2CH_2COO)_{n^3}Y^3 \quad (III)$$

   wherein $X^3$ represents a hydrogen atom, a halogen atom or a hydroxyl group, which may be identical or different to any other $X^3$ group if $n^3$ is greater than 1, $X^4$ represents a hydrogen atom, a halogen atom, a hydroxyl group or an alkyl group, $n^3$ represents an integer of from 1 to 4 and $Y^3$ represents a hydrogen atom or a uni- to tetravalent metal atom;
   (iv) 1,4-butanediol; and
   (v) γ-butyrolactone.

6. A process according to claim 5 wherein the copolyester is as defined in claim 2 and the carbon source is a compound of formula (I).

7. A process according to claim 5 wherein the copolyester is as defined in claim 3 and the carbon source is a compound of formula (II), 1,4-butanediol or γ-butyrolactone.

8. A process according to claim 5 wherein the copolyester is as defined in claim 4 and the carbon source is a compound of formula (II) and a compound of formula (III).

**Patentansprüche**

1. Copolyester, enthaltend 3-Hydroxybutyrat-Einheiten und Einheiten, ausgewählt unter:
(1) 3-Hydroxyvalerat-Einheiten und 5-Hydroxyvalerat-Einheiten;
(2) 4-Hydroxybutyrat-Einheiten; und
(3) 4-Hydroxybutyrat-Einheiten und 3-Hydroxyvalerat-Einheiten;
wobei die Grundviskosität des Copolyesters, gemessen in Chloroform bei 30 °C, im Bereich von 0,4 bis 10,0 dl/g liegt.

2. Copolyester nach Anspruch 1, der von 2 bis 50 Mol-% 3-Hydroxybutyrat-Einheiten,von 3 bis 95 Mol-% 3-Hydroxyvalerat-Einheiten und von 3 bis 90 Mol-% 5-Hydroxyvalerat-Einheiten umfaßt.

3. Copolyester nach Anspruch 1, der von 40 bis 97 Mol-% 3-Hydroxybutyrat-Einheitenund von 3 bis 60 Mol-% 4-Hydroxybutyrat-Einheiten umfaßt.

4. Copolyester nach Anspruch 1, der von 10 bis 90 Mol-% 3-Hydroxybutyrat-Einheiten, von 3 bis 60 Mol-% 4-Hydroxybutyrat-Einheiten und von 5 bis 87 Mol-% 3-Hydroxyvalerat-Einheiten umfaßt.

5. Verfahren zur Herstellung eines Copolyesters wie in Anspruch 1 definiert, wobei das Verfahren umfaßt: Kultivieren von Alcaligenes eutrophus in einer begrenzten Quelle für Stickstoff und/Phosphor und in Gegenwart einer Kohlenstoffquelle, um den Copolyester in Alcaligenes eutrophus zu bilden und anzusammeln, wobei die Kohlenstoffquelle ausgewählt ist unter:
(i) einer Verbindung der Formel (I):

$$(CH_2X^1CH_2CH_2CH_2COO)_{n^1}Y^1 \qquad (I)$$

worin $X^1$ eine Hydroxygruppe oder ein Halogenatom ist, das gleich oder verschieden zu einer anderen $X^1$-Gruppe ist, wenn $n^1$ größer als 1 ist, wobei $n^1$ eine ganze Zahl von 1 bis 4 darstellt und $Y^1$ ist ein Wasserstoffatom oder ein ein- bis vierwertiges Metallatom;
(ii) eine Verbindung der Formel (II):

$$(CH_2X^2CH_2CH_2COO)_{n^2}Y^2 \qquad (II)$$

worin $X^2$ eine Hydroxygruppe oder ein Halogenatom ist, die gleich oder verschieden sein können zu einer anderen $X^2$-Gruppe, wenn $n^2$ größer als 1 ist, wobei $n^2$ eine ganze Zahl von 1 bis 4 ist und $Y^2$ ist ein Wasserstoffatom oder ein einbis vierwertiges Metallatom;
(iii) eine Verbindung der Formel (II) wie oben definiert und eine Verbindung der Formel (III):

$$(CH_2X^3CHX^4CH_2CH_2COO)_{n^3}Y^3 \qquad (III)$$

worin $X^3$ ein Wasserstoffatom, ein Halogenatom oder eine Hydroxygruppe ist, die gleich oder verschieden sein können zu einer beliebigen anderen $X^3$-Gruppe, wenn $n^3$ größer als 1 ist, $X^4$ ist ein Wasserstoffatom, ein Halogenatom, eine Hydroxygruppe oder eine Alkylgruppe, $n^3$ ist eine ganze Zahl von 1 bis 4 und $Y^3$ ist ein Wasserstoffatom oder ein ein- bis vierwertiges Metallatom;
(iv) 1,4-Butandiol; und
(v) $\gamma$-Butyrolacton.

6. Verfahren nach Anspruch 5, worin der Copolyester wie in Anspruch 2 definiert ist und die Kohlenstoffquelle eine Verbindung der Formel (I) ist.

7. Verfahren nach Anspruch 5, worin der Copolyester wie in Anspruch 3 definiert ist, und die Kohlenstoffquelle eine Verbindung der Formel (II), 1,4-Butandiol oder $\gamma$-Butyrolacton.

8. Verfahren nach Anspruch 5, worin der Copolyester wie in Anspruch 4 definiert ist, und die Kohlenstoffquelle eine Verbindung der Formel (II) und eine Verbindung der Formel (III) ist.

**Revendications**

1. Copolyester comprenant des unités 3-hydroxybutyrate et des unités choisies parmi :
   (1) des unités 3-hydroxyvalérate et des unités 5-hydroxyvalérate;
   (2) des unités 4-hydroybutyrate; et
   (3) des unités 4-hydroxybutyrate et des unités 3-hydroxyvalérate;
   la viscosité intrinsèque du copolyester mesurée dans le chloroforme à 30 ° C étant de 0,4 à 10,0 dl/g.

2. Copolyester selon la revendication 1, qui comprend de 2 à 50 moles % d'unités 3-hydroxybutyrate, de 3 à 95 moles % d'unités 3-hydroxyvalérate et de 3 à 90 moles % d'unités 5-hydroxyvalérate.

3. Copolyester selon la revendication 1, qui comprend de 40 à 97 moles % d'unités 3-hydroxybutyrate et de 3 à 60 moles % d'unités 4-hydroxybutyrate.

4. Copolyester selon la revendication 1, qui comprend de 10 à 90 modes % d'unités 3-hydroxybutyrate, de 3 à 60 modes % d'unités 4-hydroxybutyrate et de 5 à 87 modes % d'unités 3-hydroxyvalérate.

5. Procédé pour la fabrication d'un copolyester tel que défini dans la revendication 1, lequel procédé comprend :
   la culture d'*Alcaligenes eutrophus* dans une source limitée d'azote et/ou de phosphore et en présence d'une source de carbone pour former et accumuler le copolyester dans l'*Alcaligenes eutrophus*, la source de
   carbone étant choisie parmi : (i) un composé de formule (I) :

$$(CH_2X^1CH_2CH_2CH_2COO)_{n^1}Y^1 \qquad (I)$$

   dans laquelle $X^1$ représente un groupe hydroxy ou un atome d'halogène, qui peut être identique ou différent à tout autre groupe $X^1$ si $n^1$ est supérieur à 1, $n^1$ représente un entier valant de 1 à 4 et $Y^1$ représente un atome d'hydrogène ou un atome de métal mono- à tétravalent.
   (ii) un composé de formule (II) :

$$(CH_2X^2CH_2CH_2COO)_{n^2}Y^2 \qquad (II)$$

   dans laquelle $X^2$ représente un groupe hydroxy ou un atome d'halogène, qui peut être identique ou différent à tout autre groupe $X^2$ si $n^1$ est supérieur à 1, $n^2$ représente un entier valant de 1 à 4 et $Y^2$ représente un atome d'hydrogène ou un atome de métal mono- à tétravalent;
   (iii) un composé de formule (II) tel que défini ci-dessus et un composé de formule (III) :

$$(CH_2X^3CHX^4CH_2CH_2COO)_{n^3}Y^3 \qquad (III)$$

   dans laquelle $X^3$ représente un atome d'hydrogène, un atome d'halogène ou un groupe hydroxy, qui peut être identique ou différent à tout autre groupe $X^3$ si $n^3$ est supérieur à 1, $X^4$ représente un atome d'hydrogène, un atome d'halogène, un groupe hydroxy ou un groupe alkyle, $n^3$ représente un entier valant de 1 à 4 et $Y^3$ représente un atome d'hydrogène ou un atome de métal mono- à tétravalent;
   (iv) le 1,4-butanediol; et
   (v) le $\gamma$-butyrolactone.

6. Procédé selon la revendication 5, dans lequel le copolyester est tel que défini dans la revendication 2 et la source de carbone est un composé de formule (I).

7. Procédé selon la revendication 5, dans lequel le copolyester est tel que défini dans la revendication 3 et la source de carbone est un composé de formule (II), le 1-4-butanediol ou le $\gamma$-butyrolactone.

8. Procédé selon la revendication 5, dans lequel le copolyester est tel que défini dans la revendication 4 et la source de carbone est un composé de formule (II) et un composé de formule (III).

Fig.1

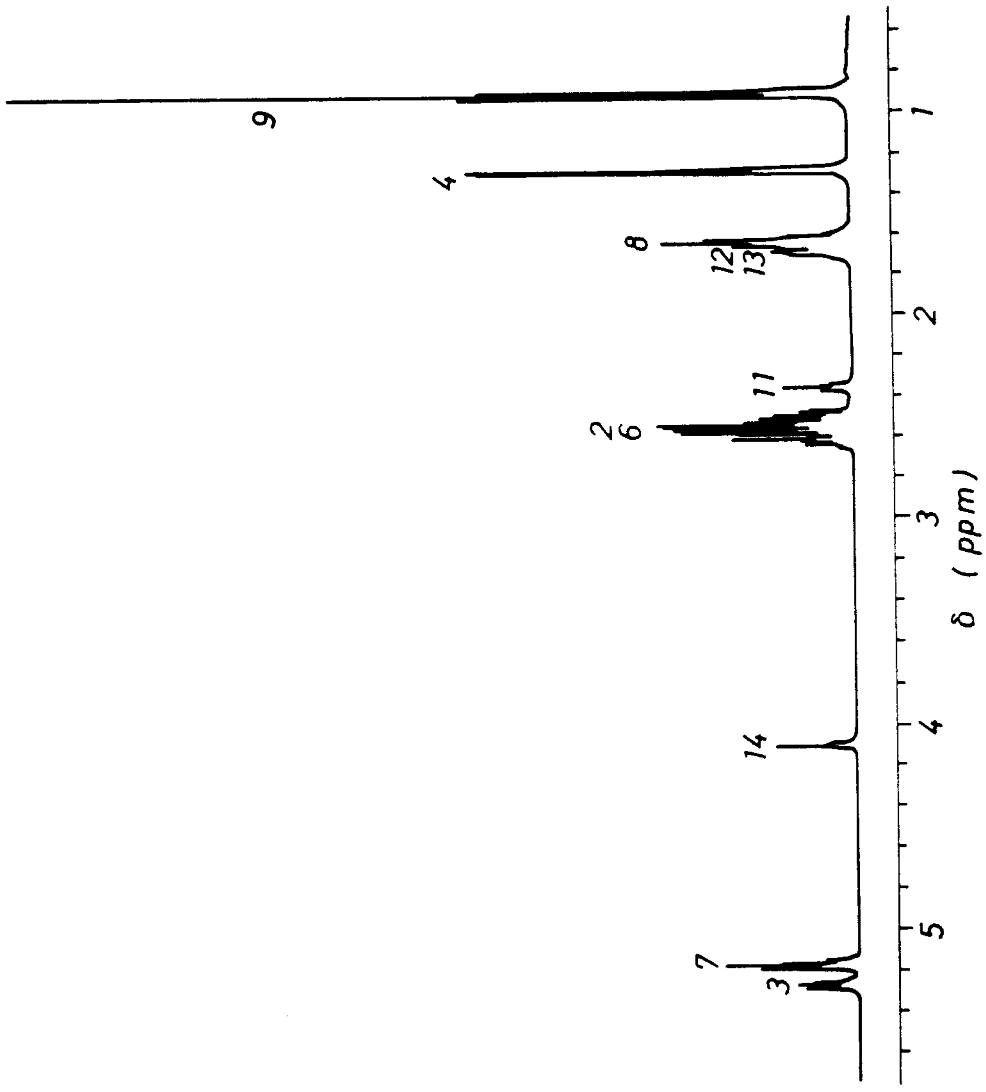

Fig. 2

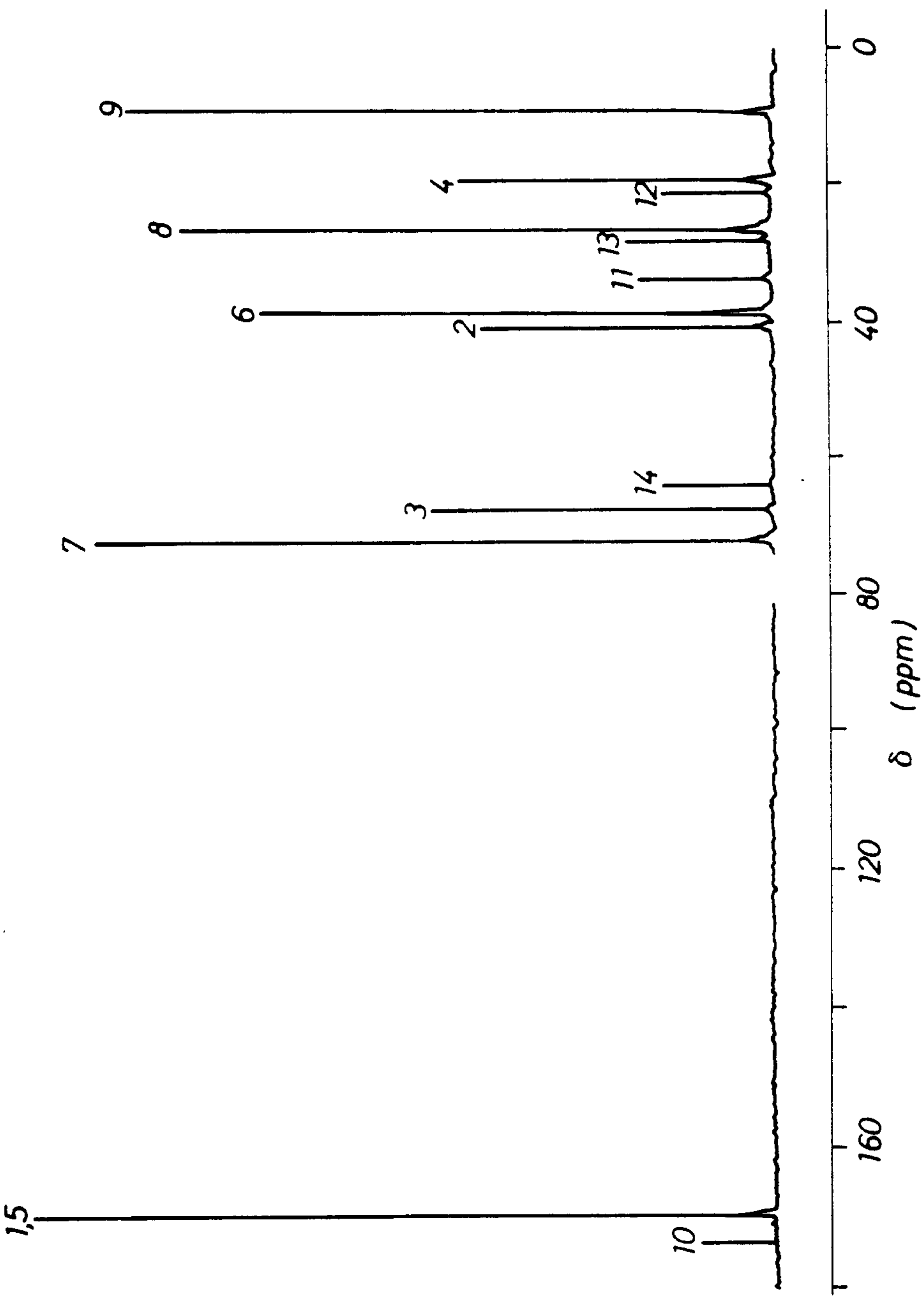

Fig. 3

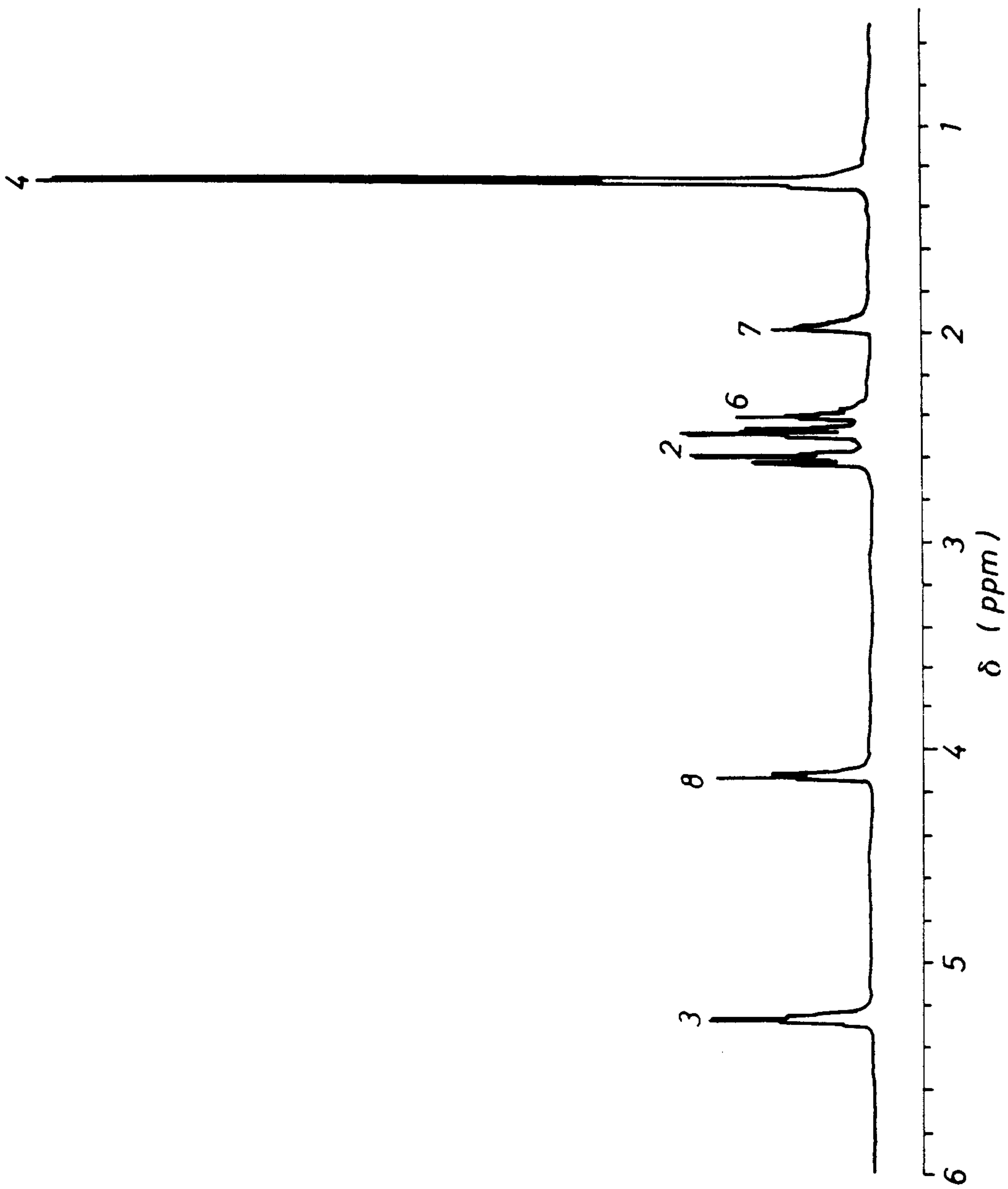

Fig. 4

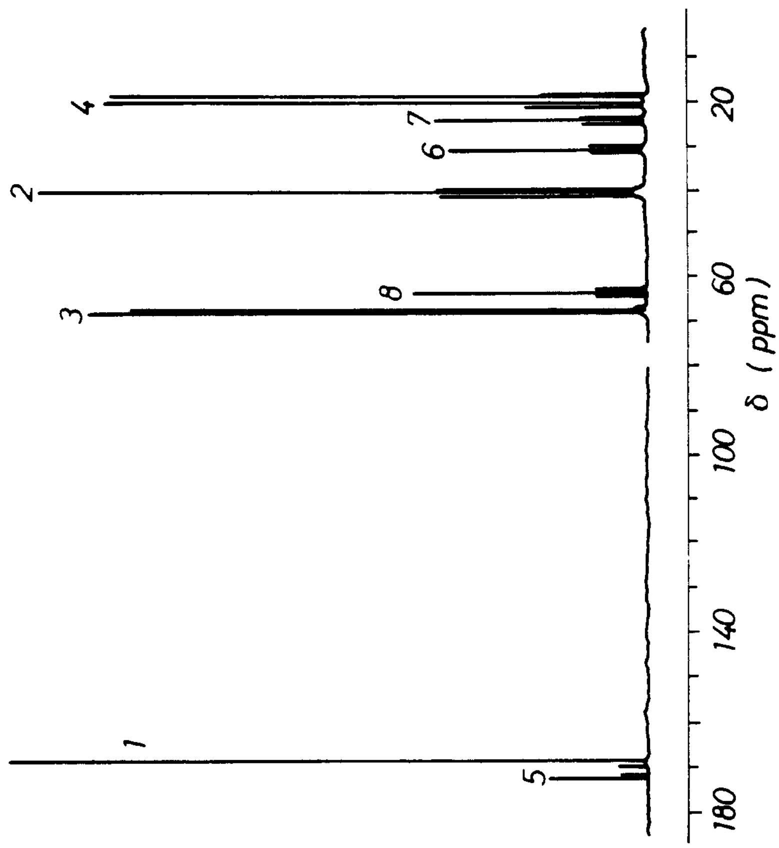

Fig.5

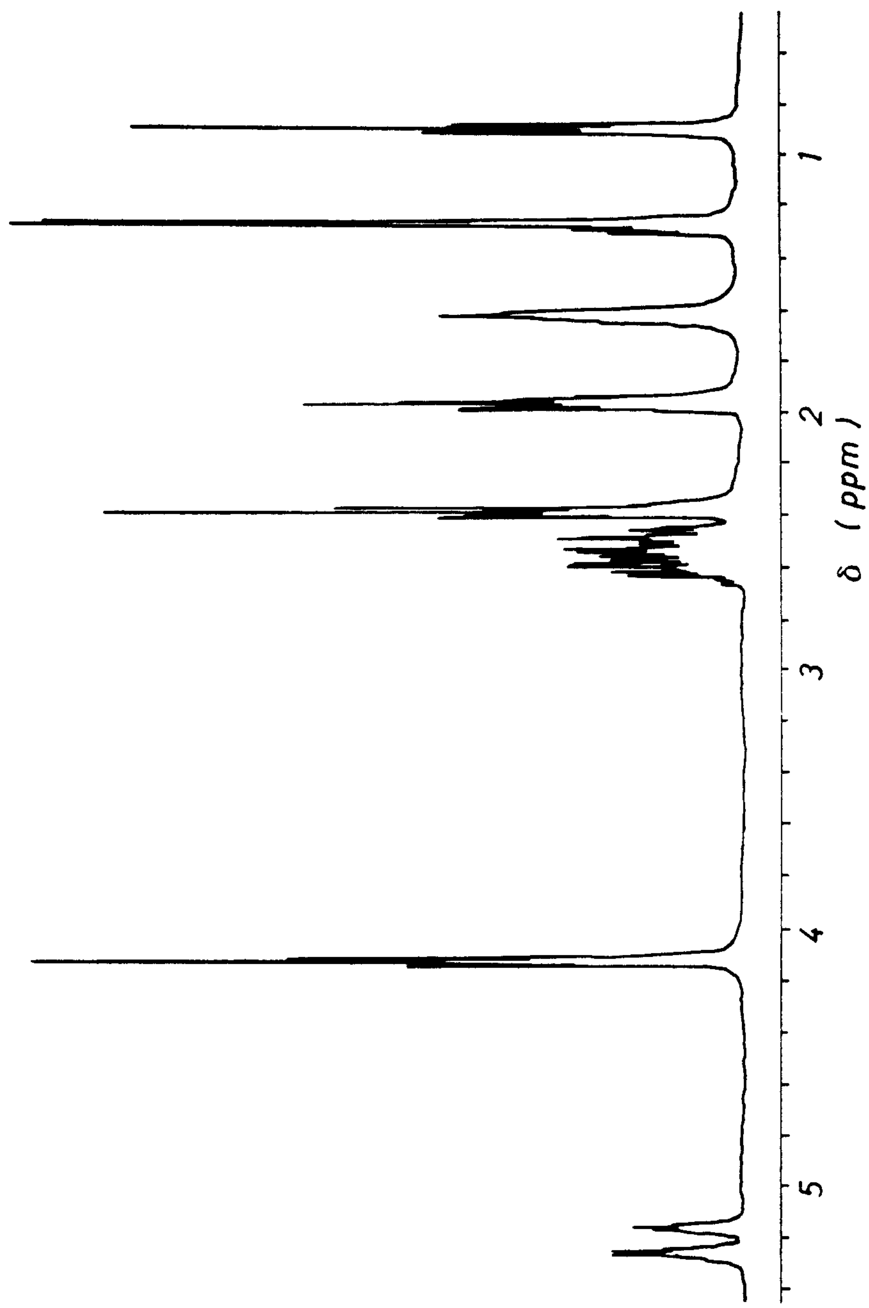

Fig. 6

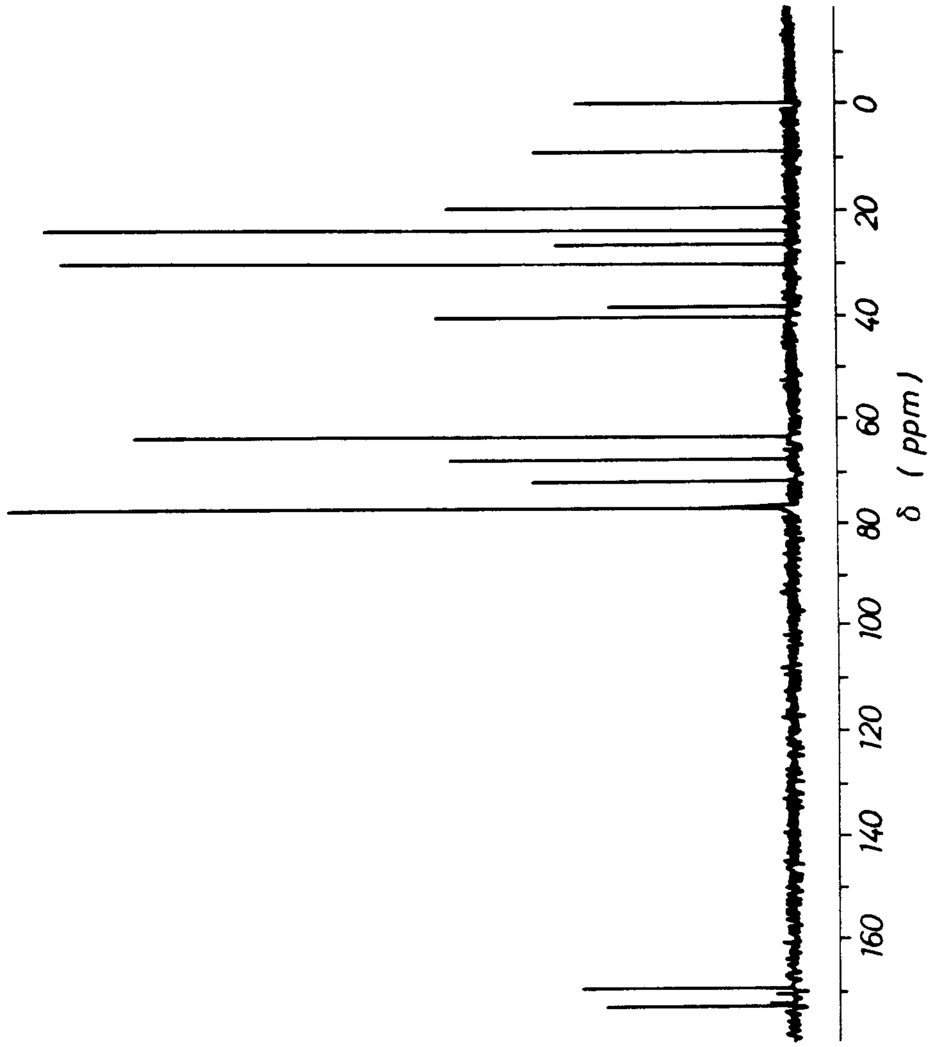

Fig. 7

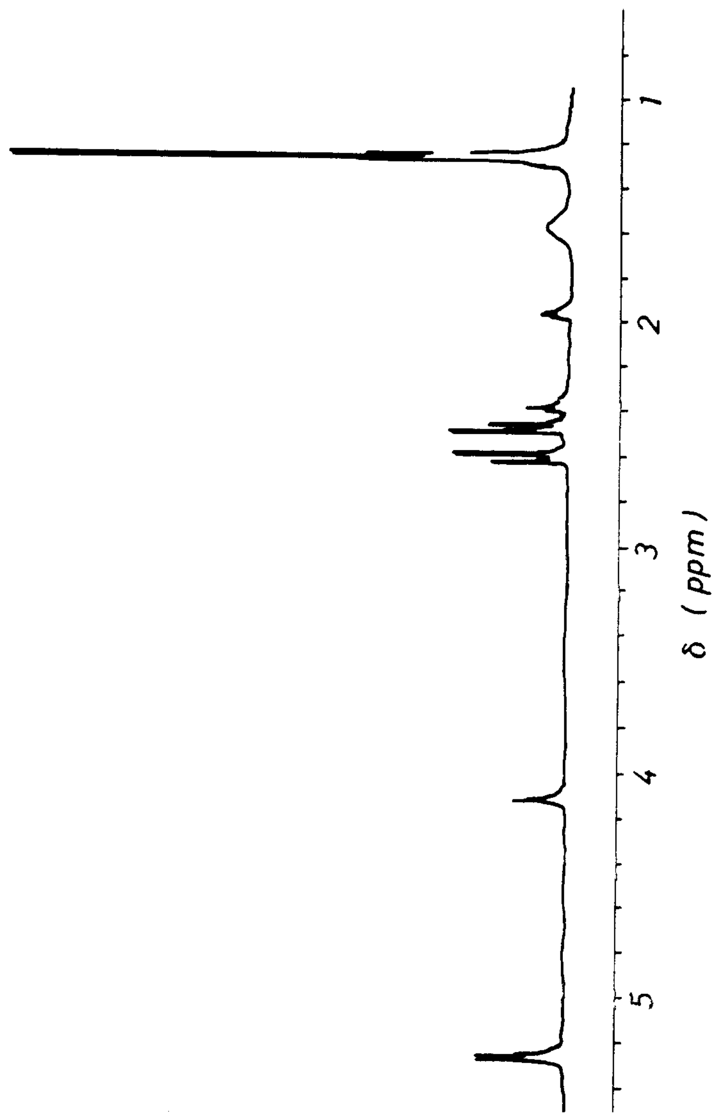

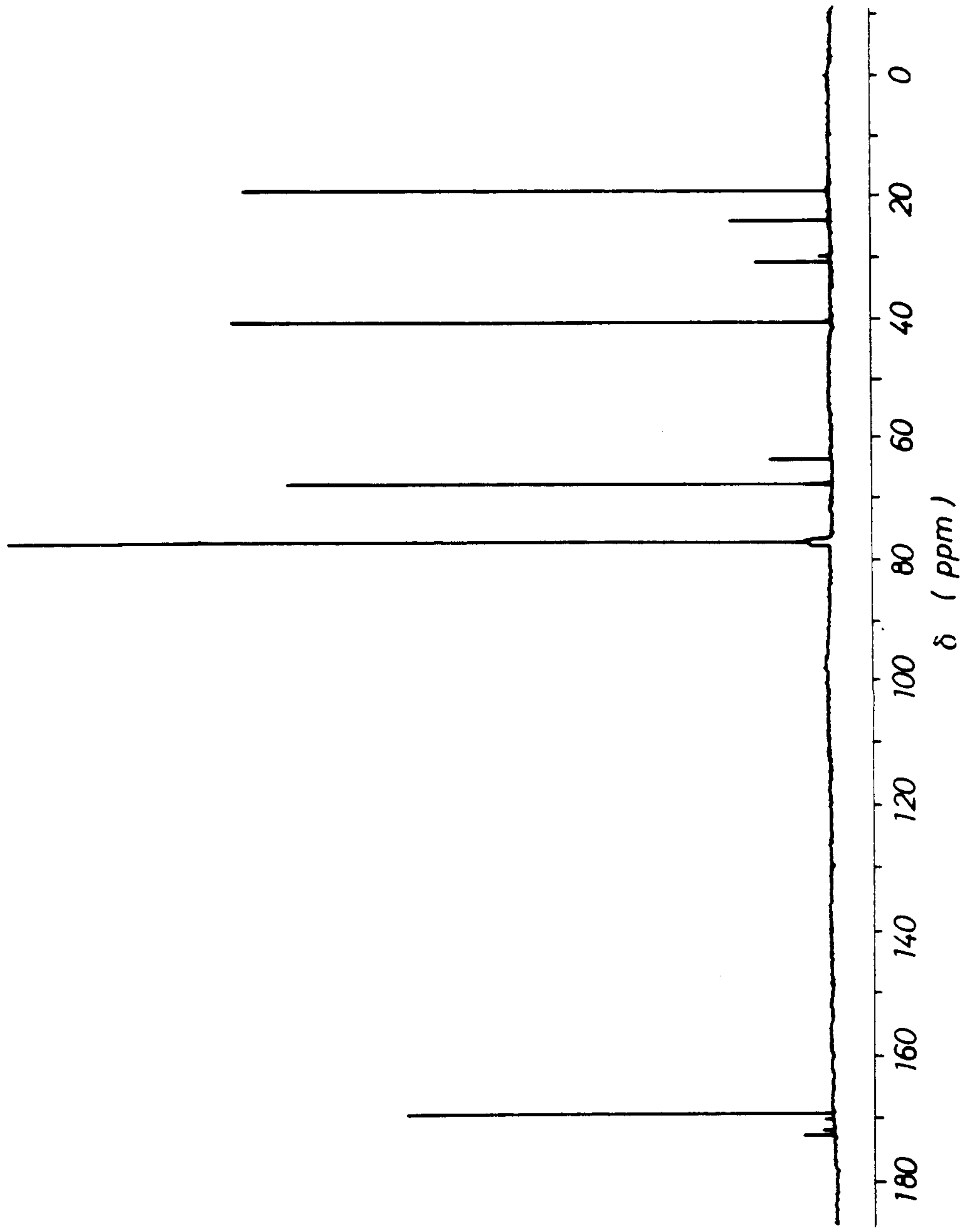


Fig. 8

Fig. 9